# EUROPÄISCHE PATENTSCHRIFT

(11) **EP 0 527 541 B1**
(45) Veröffentlichungstag und Bekanntmachung des Hinweises auf die Patenterteilung: **19.07.1995**
(21) Anmeldenummer: 92250201.8
(22) Anmeldetag: 04.08.1992
(51) Int. Cl.: A61B 1/06, A61B 1/04

(54) **Endoskop**
Endoscope
Endoscope

(30) Priorität: 08.08.1991 DE 9109924 U
(43) Veröffentlichungstag der Anmeldung: 17.02.1993
(73) Patentinhaber: Effner Biomet GmbH, D-12247 Berlin (DE)
(72) Erfinder: Anapliotis, Emmanuel, W-1000 Berlin 33 (DE); Schich, Gisbert, W-8800 Ansbach (DE)
(74) Vertreter: Christiansen, Henning, Dipl.-Ing.

(56) Entgegenhaltungen:
- DE-A- 2 637 133
- DE-A- 3 429 945
- DE-B- 2 062 951
- DE-U- 8 814 573
- US-A- 4 838 247

## Beschreibung

Die Erfindung betrifft ein Endoskop der im Oberbegriff des Anspruchs 1 angegebenen Art.

Derartige Endoskope werden zur Untersuchung von Körperhohlräumen verwendet und dienen in Form von Arthroskopen der Untersuchung und Behandlung von Gelenken, meist der Kniegelenke, in dem man den Gelenkinnenraum nach Einführung des Arthroskops betrachten kann. Vorzugsweise zur Abklärung von Meniskusverletzungen ist die Arthroskopie ein häufig angewendetes Verfahren.

Da der Gelenkinnenraum nicht nur betrachtet, sondern auch beleuchtet wird, weisen Endoskope, wie beispielsweise auch das aus der DE-U-88 14 573 bekannte Endoskop, vorzugsweise getrennte Beleuchtungs- und Beobachtungsbaugruppen auf, die ineinander steckbar sind. Dabei ist die Beobachtungsbaugruppe, die aus einem optiktragenden Schaft, einem Objektiv am in den Gelenkinnenraum einzuführenden distalen Ende des Schafts und einem Okular am proximalen Beobachtungsende des Schafts besteht, in den Lichtschaft der Beleuchtungsbaugruppe einschiebbar.

Nachteilig bei den bekannten Endoskopen ist aber, daß die Beobachtungsbaugruppe stets vollständig ersetzt werden muß, wenn eine Optik mit einem anderen Beobachtungswinkel für die weitere Untersuchung benötigt wird oder diese im Falle eines Defekts ersetzt werden muß. Insbesondere muß bei einem Wechsel der Optik stets sowohl im sterilen als auch im unsterilen Bereich manipuliert werden, so daß auch eine die jeweiligen Bereiche trennende Abdeckung jeweils erneuert werden muß.

Der Erfindung liegt die Aufgabe zugrunde, bei einem Endoskop der eingangs genannten Gattung die oben geschilderten Nachteile zu beseitigen, um ein Endoskop zu erhalten, das einerseits kostengünstig herstellbar ist und bei dem andererseits Teile ohne großen Aufwand - auch unter Operationsbedingungen - ausgetauscht werden können, wobei insbesondere die Sterilität im Operationsbereich erhalten bleiben soll.

Diese Aufgabe wird mit den kennzeichnenden Merkmalen des Anspruchs 1 gelöst.

Die Erfindung schließt die Erkenntnis ein, daß ein vorteilhaftes Endoskop geschaffen werden kann, wenn die Beobachtungsbaugruppe derart zerlegbar ist, daß die gegebenenfalls zu ersetzenden Teile leicht zugänglich und derart austauschbar sind, daß im Falle eines Defekts nur die wirklich beschädigten Teile ersetzt werden müssen.

Insbesondere müssen bei einem derartigen Austausch der Optik nur wenige Bauteile der Beobachtungsbaugruppe beim Austausch der mit einem optiktragenden Schaft verbundenen inneren Optik gelöst und manipuliert werden, wobei die Verbindung mit einer Beobachtungs- und Beleuchtungsbaugruppe grundsätzlich erhalten bleibt. Dadurch, daß der innere Optikteil als Rotationsoptik ausgeführt ist, kann durch Verdrehen dieses Teils gegenüber der feststehenden Hülse zusätzlich die Betrachtungsrichtung ausgewählt werden, wenn die Blickrichtung der Optik zu ihrer Achse geneigt ist. Die vollständige aus Rotationsoptik und Optik-Fuhrungshülse bestehende Beobachtungsbaugruppe kann in ihren äußeren Abmaßen so gestaltet werden, daß sie mit einteiligen Beobachtungsbaugruppen anderer Hersteller austauschbar ist, so daß auch insoweit Kompatibilität besteht.

Durch Bereithalten einer Anzahl von Rotationsoptiken mit unterschiedlichen Beobachtungswinkeln (Winkelbereich und "Seitblickwinkel" in Bezug auf die Längsachse) besteht ein großes Anwendungsspektrum für das erfdingungsgemäße Endoskop. Hierbei ist es ein zusätzlicher Vorteil, wenn eine mit der Beobachtungsbaugruppe verbundene Videoeinheit, insbesondere eine Kamera, nicht bei jedem Tauschvorgang gelöst und erneut angebracht werden muß. Dabei ist vor allem von Bedeutung, daß die Kamera sich stets unter einer sterilen Folienabdeckung befindet, wobei die Verbindungsstelle zwischen der Aufzeichnungseinheit und der Beobachtungsbaugruppe innerhalb des Sterilbereichs der Folienabdeckung gelegen ist, so daß ein erneutes Sterilisieren der nicht abgedeckten Bereiche und der Folienabdeckung nach Wiederherstellung der Verbindung notwendig ist.

Damit ist bei einem Endoskopsystem, welches unterschiedliche Betrachtungswinkel ermöglicht, lediglich die Bereithaltung von unterschiedlichen - aber in ihren äußeren Anschlußmaßen einheitlichen - Rotationsoptiken, erforderlich, welche jeweils einzeln in die mit der Beleuchtungsbaugruppe verbundene Optikführungshülse der Beobachtungsbaugruppe einsteckbar sind. Die Anschaffungskosten eines derartigen Endoskops mit unterschiedlichen Optiken verringern sich dadurch erheblich und die effektive Einsatzzeit eines Instruments kann stark, aufgrund der relativ kurzen Austausch- bzw. Reparaturzeit, gesteigert werden.

Beim Wechsel der Optik besteht eine Trennstelle im sterilen Bereich, die für das ebenfalls sterile Operationspersonal zugänglich ist. Eine angeschlossene Video-Kamera verbleibt auf der nicht-sterilen Seite unter einem Überzug, so daß bei einem Optikwechsel eine Neusterilisation bzw. ein Ersetzen des Überzugs der Kamera entfällt, was jeweils mit einer umständlichen und zeitaufwendigen Handhabung verbunden wäre, da dabei in den sterilen Bereich von unsterilen Personen eingegriffen werden müßte, so daß stets die Gefahr der Kontaminierung auch steriler Personen bestünde, welche sich daraufhin erst wieder einer aufwendigen Reinigungsprozedur unterziehen müßten.

Die erfindungsgemäße Beobachtungsbaugruppe besteht aus einer mit einem optiktragenden Schaft verbundenen Rotationsoptik, die innerhalb einer Optikführungshülse angeordnet ist. Die Optikführungshülse ist proximal, also okularseitig, mit einem abschraubbaren Optiktrichter verbunden und weist Verbindungselemente auf, mittels denen die Beleuchtungsbaugruppe und die Beobachtungsbaugruppe miteinander verbindbar sind. Dadurch, daß nur die Optikführungshülse mit der Beobachtungsbaugruppe fest verbunden ist, ist die mit dem optiktragenden Schaft verbundene Rotationsoptik beim Abschrauben des Optiktrichters durch die damit entstehende Öffnung leicht zugänglich und austauschbar.

Bei einer mit dem Optiktrichter mittelbar oder über einen Anpassungsstück verbundenen Videoeinheit, ist der Optiktrichter derart ausgebildet, daß dessen Verbindungen sowohl mit der Optikführungshülse als auch mit der Aufzeichnungseinheit lösbar sind, so daß nach dem Abschrauben des Optiktrichters nicht nur die sterile Beobachtungsbaugruppe von der nicht sterilen Kamera getrennt wird, sondern auch das Innere der Beobachtungsbaugruppe für einen Optikaustausch zugänglich ist.

Die Verbindung zwischen Optiktrichter und Optikführungshülse ist bevorzugt als Schraubverbindung ausgebildet, während die Verbindung zwischen Optiktrichter und Aufzeichnungseinheit bevorzugt als Klemm- oder Steckverbindung ausgebildet ist, um die beidseitige Abtrennung des Optiktrichters zu erleichtern. Dabei wird zuerst die Klemm- oder Steckverbindung zwischen Aufzeichnungseinheit und Optiktrichter gelöst, in dem die Aufzeichnungseinheit vom Endoskop entfernt wird. Hierbei wird nur die Stellung der Videoeinheit und nicht die des Endoskops geändert, welches bei einem im Körper eingesetzten Endoskop eine wichtige Erfordernis ist, da ein unkontolliertes weiteres Eindringen des Endoskops in die Körperhöhle, aufgrund einer Verletzungsgefahr des Patienten, vermieden werden muß. Danach kann der jetzt gut zugängliche Optiktrichter von der Optikführungshülse abgeschraubt werden, und die in der Hülse liegende mit dem optiktragenden Schaft verbundene Rotationsoptik aus der Hülse entnommen werden.

Durch die Trennmöglichkeit von Optiktrichter und der an dem entsprechenden Adapter mit einer sterilen Abdeckung versehenen Aufzeichnungseinheit in günstiger Weise möglich, daß die sterile Abdeckung auch bei einer schnellen An- und Abkopplung des Optiktrichters an die Aufzeichnungseinheit nicht entfernt werden muß und auch nicht beschädigt wird.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein vorteilhaftes Ausführungsbeispiel der zusammengebauten Beobachtungsbaugruppe als Teil des erfindungsgemäßen Endoskops,
Figur 2 eine weitere Darstellung der Beobachtungsbaugruppe gemäß Figur 1, wobei die Rotationsoptik teilweise aus der Optikführungshülse herausgezogen ist,
Figur 3 ein vorteilhaftes Ausführungsbeispiel der Beleuchtungsbaugruppe als Teil des erfindungsgemäßen Endoskops,
Figur 4 das erfindungsgemäße Endoskop in montiertem Zustand - teilweise im Schnitt dargestellt- sowie
Figur 5 eine vergrößerte Darstellung eines Optiktrichters als okularseitiges Element der Beobachtungsbaugruppe, der mit einer Anzeigeeinheit und mit der Beobachtungsbaugruppe verbunden ist.

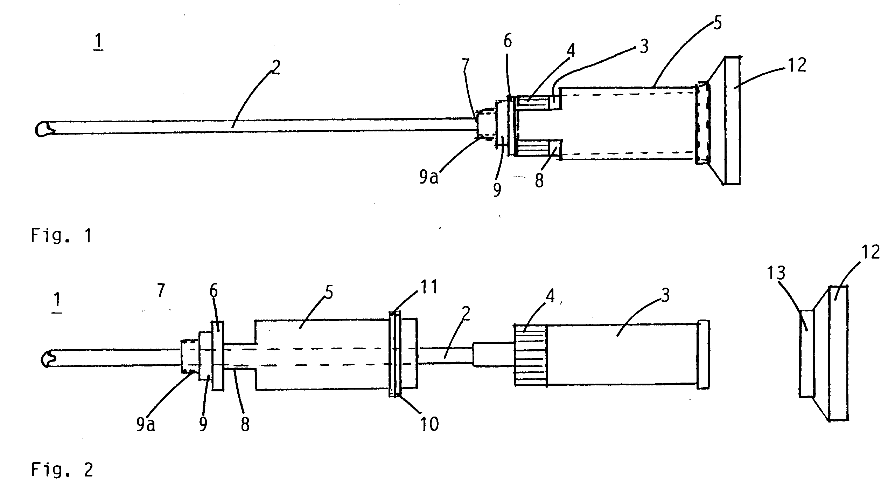

Die nachfolgende Beschreibung erfolgt unter gleichzeitiger Bezugnahme auf die Figuren 1 und 2, wobei Figur 1 die in Funktionsstellung zusammengesetzte Beobachtungsbaugruppe 1 und Figur 2 die in ihre einzelnen Teilen zerlegte Beobachtungsbaugruppe 1 zeigt.

Die Beobachtungsbaugruppe 1 besteht aus einer einen optiktragenden Schaft 2 aufweisenden Rotationsoptik 3, einer Optikführungshülse 5 und einem okularseitigen Optiktrichter 12. Die Rotationsoptik 3 ist im Bereich des optiktragenden Schafts 2 stabförmig ausgestaltet und enthält die für die Bilderzeugung und -weiterleitung notwendigen Linsen. Sie bildet eine abgeschlossene Einheit und ist in Figur 2 teilweise aus der Optikführungshülse 5 herausgezogen wiedergegeben. Die Rotationsoptik 3 weist einen zur manuellen Betätigung gerändelt ausgeführten Rotationsring 4 auf, mittels dessen die Optik innerhalb der Hülse 5 verdrehbar ist (vgl. Figur 1), wenn diese in ihrer montierten Position durch die weiteren Teile des Endoskops - wie unten beschrieben - festgehalten ist. Die Wandung der Optikführungshülse 5 weist zu diesem Zweck mindestens eine entsprechend de Aussparung 8 auf, durch die der rändelförmig ausgestaltete Rotationsring 4 der Rotationsoptik 3 von außen erreichbar und von Hand drehbar ist. Mit der Rotationsoptik kann die Blickrichtung des Objektivs durch Verdrehen der Optik um ihre Längsachse geändert werden, wenn dieses an der Objektseite eine von der Längsachse abweichende Blickrichtung aufweist. Zuätzlich kann auch der Blickwinkel durch Auswahl einer einen entsprechenden Blickwinkel aufweisenden Optik verändert werden. Entsprechendes gilt für den zu überblickenden Winkelbereich.

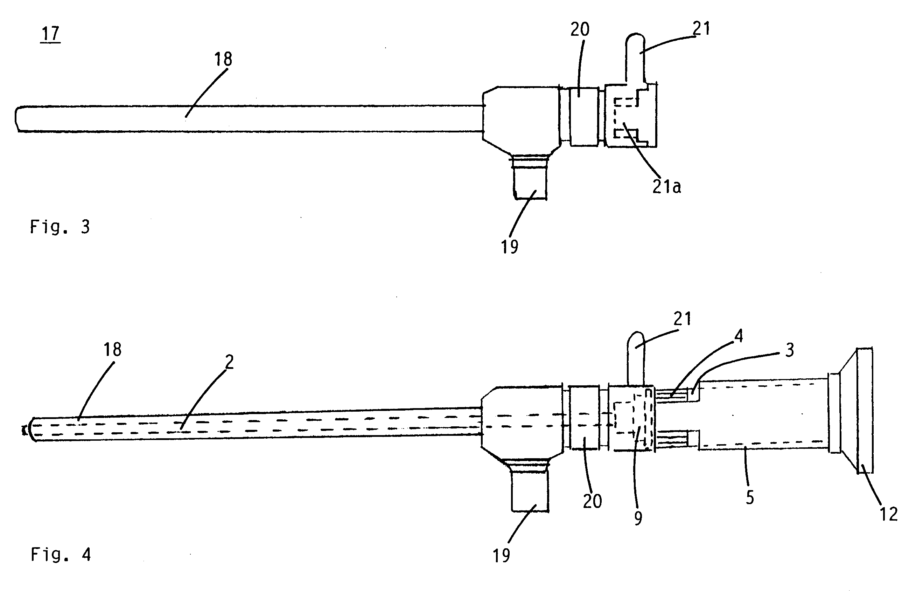

Die Optikführungshülse 5 weist an ihrem distalen Ende 6 eine eine Öffnung 7 auf, durch die der optiktragende Schaft 2 der Rotationsoptik 3 beim Zusammenbau der Beobachtungsbaugruppe 1 eingesetzt wird. Zudem weist die Optikführungshülse 5 eine Kupplungsvorrichtung 9 auf, mit der sie mit einer Beleuchtungsbaugruppe 17, die anhand von Figur 3 näher erläutert wird, verbunden wird.

Die Kupplungsvorrichtung 9 der Optikführungshülse ist vorzugsweise als ein ein Außengewinde aufweisender Gewindeansatz 9a ausgebildet, der in eine entsprechende als eine mit einem Innengewinde versehene Bohrung 21a der Beleuchtungsbaugruppe 17 gemäß Figur 3 einschraubbar ist. Die Kupplungsvorrichtung 9, 21a kann aber auch andersartig, beispielsweise als Steck- oder Bajonettverbindung, ausgestaltet sein. Die Optikführungshülse 5 und die Beleuchtungseinheit können damit zum Austausch der Rotationsoptik miteinander verbunden bleiben, so daß letztere eineseits drehbar gelagert, andererseits aber auf einfache Weise austauschbar ist, ohne daß die Beleuchtungseinheit abgenommen werden müßte. Mittels einer Arretierungsvorrichtung 21, die insbesondere als radial verschieblicher Stift, der mit seinem innen gelegenen Ende in eine entsprechende - nicht dargestellte - Aussparung eingreift oder in sonstiger Weise eine Rotationshemmung bildet, kann die Rotationsoptik gegebenenfalls arretiert werden, so daß die Blickrichtung unverändert bleibt.

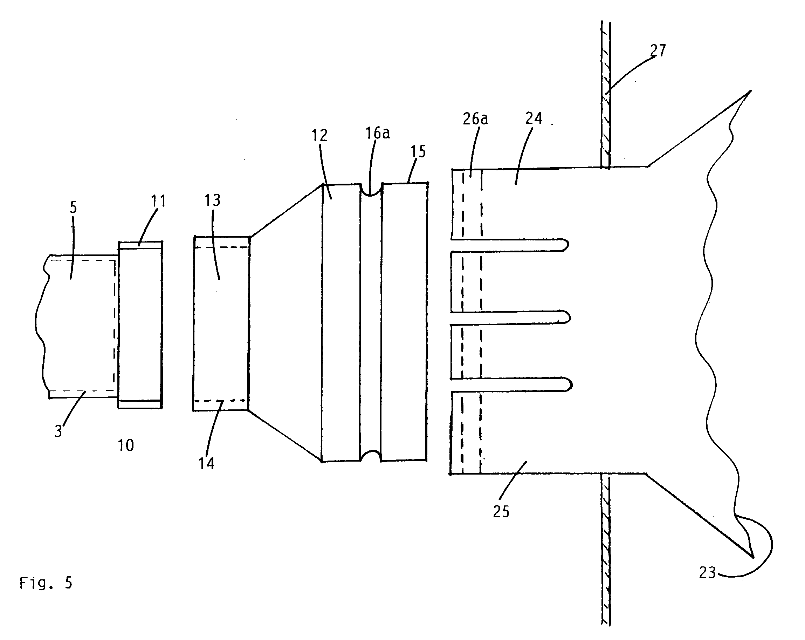

Okularseitig, ist ein Optiktrichter 12 mit der Optikführungshülse 5 verbunden, wobei die Funktion des Optiktrichters 12 anhand von Figur 5 weiter unten noch ausführlich erläutert werden wird. Beim Zusammenbau der Beobachtungsbaugruppe 1 wird die Optikführungshülse 5 in den optiktragenden Schaft 2 bis zum Anschlag der Rotationsoptik 3 vom okularseitigen Ende 6 der Optikführungshülse 5 her eingeführt. Anschließend wird der Optiktrichter 12 okularseitig auf das Ende der Optikführungshülse 5 aufgeschraubt. Damit bildet die Optikführungshülse 5 eine Umhüllung für die Rotationsoptik, deren okularseitige Öffnung durch den Optiktrichter verschlossen wird.

Die Optikführungshülse 5 bildet damit eine relativ zur Optik feststehende Umkapselung für den manuell betätigbaren Rotationsring 4 der Rotationsoptik mit mindestens einer den Zugang zu dem Rotationsring freigebenden Aussparung 8. Die feststehende Optikführungshülse weist damit Anschlußmittel für eine den optiktragenden Schaft 2 der Rotationsoptik umgebendes Schutzrohr (hier: Beleuchtungseinheit 17) einerseits und Kameraanschluß (hier: Optiktrichter 12) auf, so daß die Rotationsoptik in der so gebildeten "Kammer" manuell zugänglich rotierbar ist, während die äußeren Komponenten bis hin zum Kameraanschluß feststehend ausgebildet sind.

Die in Figur 3 dargestellte Beleuchtungsbaugruppe 17, in die die Beobachtungsbaugruppe einfügbar ist, besteht aus einer in den Gelenkinnenraum einzuführenden Kanüle 18 mit einem daran angeschlossenen Beleuchtungsstutzen 19, der zum Anschluß von selbsttragenden oder flexiblen Lichtleitkabeln dient zwecks Lichtzuführung dient, einem der den Zu- bzw. Abfluß von Spülsubstanz bzw. abgesaugten Flüssigkeit regelnden Baugruppe 20 der Saug- bzw. Spüleinrichtung sowie einer zweiten Kupplungsvorrichtung 21 zum Ankuppeln der Beleuchtungsbaugruppe 17 an die an der Optikführungshülse 5 der Beobachtungsbaugruppe 1 angebrachte ersten Kupplungsvorrichtung 9. Die Kupplungsvorrichtung weist - wie erwähnt - zusätzliche Mittel zur Verriegelung der in der Optikführungshülse 5 drehbar gelagerten Rotationsoptik 3 auf.

Das in Figur 4 dargestellte Ausführungsbeispiel des erfindungsgemäßen Endoskopn ist durch das Einführen des optiktragenden Schafts 2 in die Kanüle 18 und mittels der Ankopplung der Beleuchtungs- 17 an die Beobachtungsbaugruppe 1 mit nur wenigen Handgriffen montierbar. Bei einem Ausbau der Rotationsoptik 3, sei es weil sie beschädigt oder weil eine andere Rotationsoptik 3 mit einem anderen Blickwinkel benötigt wird, muß nur der Optiktrichter 12 von der Optikführungshülse 5 abgeschraubt werden, um die bereits mittels der Kupplungsvorrichtung 21 entriegelte Rotationsoptik 3 aus dem Endoskop entnehmen zu können. Die neue Rotationsoptik 3 wird in umgekehrter Reihenfolge wieder eingesetzt.

Der Aufbau des erfindungsgemäßen Optiktrichters 12 und seine Verbindung mit der Aufzeichnungseinheit 23 und der Optikführungshülse 5 wird anhand der Figur 5 erläutert.

Der Optiktrichter 12, der als Augenmuschel oder Kameraanschluß dient und die Rotationsoptik 3 vor Beschädigungen schützt, ist am distalen Ende mit einem Ansatz 13 versehen, der ein Innengewinde 14 aufweist, und der auf die Optikführungshülse 5, die ein entsprechendes Außengewinde 11 aufweist, aufschraubbar ist. Der Optiktrichter 12 kann auch andersartig, beispielsweise steck- bzw. bayonettverschlußartig, mit der Optikführungshülse 5 verbindbar sein. Da der Optiktrichter 12 des Endoskops zudem mit einer Aufzeichnungseinheit 23 verbindbar ausgebildet ist, weist dieser an seiner radialen Außenoberfläche 15 eine Ringnut 16a auf, und der Ansatzstutzen 24, der fest mit der Aufzeichnungseinheit 23 verbunden ist und an dem eine den sterilen (objektivseitigen) Bereich von der unsterilen (die Videokamera aufweisenden) Seite trennende Abdeckung 27 befestigt ist, weist an seiner Innenseite 25 eine entsprechende Ringwulst 26b auf und ist bei einer bevorzugten Weiterbildung in Längsrichtung geschlitzt, so daß das Einführen des Optiktrichters 12 in den Ansatzstutzen 24 erleichtert wird. Alternativ kann der Optiktrichter 12 an seiner Außenoberfläche 15 auch statt dessen eine Ringwulst 16b und der Ansatzstutzen 24 eine entsprechende Ringnut 26a aufweisen. Diese Variante ist in den Figuren nicht dargestellt.

Es ist ersichtlich, daß zum Auswechseln der Rotationsoptik die Abdeckung 27 mit dem Kameraansatz 24 verbunden bleiben kann, so daß keinerlei Handhabungen also keinerlei Handhabungen im unsterilen Bereich erforderlich sind, da die Öffnung zum Einsetzen der Rotationsoptik 3 in die diese aufnehmende Optikführungshülse 5 auschließlich auf der sterilen Seite gelegen ist. Damit braucht insbesondere auch nicht bei jedem Objektivwechsel eine Abdeckung 27 bereitgestellt zu werden.

Bei dem erfindungsgemäßen Endoskop bildet damit die Optikführungshülse (5) eine im wesentlichen abgeschlossene Kammer, in der ein Rotationsring (4) als manuelles Betätigungsmittel für ein Verdrehen der Rotationsoptik (3) von außen her zugänglich angeordnet ist, wobei die in bezug auf die Rotationsoptik feststehende Optikführungshülse an ihrem einen Ende Anschlußmittel für den, ebenfalls feststehenden, einen Videoadapter bildenden, Ansatzstutzen (24) und an ihrem anderen Ende eine Hülse zum Schutz des optiktragenden Schafts (2) der Rotationsoptik (3), oder Anschlußmittel dafür, aufweist.

## Patentansprüche

1. Endoskop, insbesondere Arthroskop, bestehend aus einer Beleuchtungs- (17) und einer Beobachtungsbaugruppe (1) die miteinander verbindbar sind, wobei die Beobachtungsbaugruppe (1) einen optiktragenden Schaft (2) aufweist, der in das Innere einer Kanüle (18) der Beleuchtungsbaugruppe (17) einschiebbar ist,
**dadurch gekennzeichnet,**
daß der optiktragende Schaft (2) Teil einer Rotationsoptik (3) ist, die von einer Optikführungshülse (5) umgeben ist, wobei die Rotationsoptik (3) innerhalb der Optikführungshülse (5) dreh- bzw. verschiebbar gelagert ist, und
daß die Optikführungshülse (5) an einem Ende eine Kupplungsvorrichtung (9), über die sie mit der Beleuchtungsbaugruppe (17) verbindbar ist, und am anderen Ende einen abnehmbaren okularseitigen Optiktrichter (12) aufweist.

2. Endoskop nach Anspruch 1, **dadurch gekennzeichnet,** daß die Kupplungsvorrichtung (9) als Schraubverbindung ausgebildet ist.

3. Endoskop nach Anspruch 2, **dadurch gekennzeichnet,** daß die Optikführungshülse (5) einen Gewindeanschluß (10) mit Außengewinde (11) für den Optiktrichter (12) und dieser widerum einen Ansatz (13) mit einem entsprechenden Innengewinde (14) aufweist.

4. Endoskop nach Anspruch 3, **dadurch gekennzeichnet,** daß der Optiktrichter (12) und ein Ansatzstutzen (24) einer Aufzeichnungseinheit (23) steck- und/oder klemmbar miteinander verbindbar sind.

5. Endoskop nach Anspruch 4, **dadurch gekennzeichnet,** daß der Optiktrichter (12) an seiner Außenfläche (15) mit einer Ringnut (16a) oder Ringwulst (16b) versehen ist und in den Ansatzstutzen (24), der an seiner Innenfläche (25) eine entsprechende (Gegen-)Ringwulst (26b) bzw. -nut (26a) aufweist, einsteck- oder -schnappbar ist.

6. Endoskop nach Anspruch 5, **dadurch gekennzeichnet,** daß eine die Aufzeichnungseinheit (23) umhüllende sterile Abdeckung (27) am einen Adapter bildenden Ansatzstutzen (24) befestigbar ist, wobei die Verbindungsstelle zwischen Ansatzstutzen (24) und Optiktrichter (12) außerhalb der Abdeckung (27) gelegen ist.

7. Endoskop nach einem der vorangehenden Ansprüche, **dadurch gekennzeichnet,** daß die Optikführungshülse (5) eine im wesentlichen abgeschlossene Kammer bildet, in der ein Rotationsring (4) als manuelles Betätigungsmittel für ein Verdrehen der Rotationsoptik (3) von außen her zugänglich angeordnet ist, wobei die in bezug auf die Rotationsoptik (3) feststehende Optikführungshülse (5) an ihrem einen Ende Anschlußmittel für den, ebenfalls feststehenden, einen Videoadapter bildenden, Ansatzstutzen (24) und an ihrem anderen Ende eine Hülse zum Schutz des optiktragenden Schafts (2) der Rotationsoptik (3), oder Anschlußmittel dafür, aufweist.

8. Endoskop nach Anspruch 7, **dadurch gekennzeichnet,** daß die relative Drehlage der Rotationsoptik (3) zur Optikführungshülse (5) mittels eines Rotationsrings (4) verstellbar ist.

9. Endoskop nach Anspruch 8, **dadurch gekennzeichnet,** daß die Optikführungshülse (5) in ihrer Wandung mindestens eine Aussparung (8) aufweist, durch die der - insbesondere als Rändelring ausgebildete - Rotationsring (4) der Rotationsoptik (3) betätigbar ist.

10. Endoskop nach einem der vorangehenden Ansprüche, **dadurch gekennzeichnet,** daß der Optiktrichter (12) eine Entnahmeöffnung der Optikführungshülse (5) verschließt, durch die hindurch die Rotationsoptik (3) aus der Optikführungshülse (5) entnehmbar ist.

11. Endoskop nach einem der vorangehenden Ansprüche, bestehend aus einer Beleuchtungs- (17) und einer Beobachtungsbaugruppe (1) die relativ zueinander arretierbar sind, wobei die Beobachtungsbaugruppe (1) einen optiktragenden Schaft (2) aufweist, der in das Innere einer Kanüle (18) der Beleuchtungsbaugruppe (17) einschiebbar ist, **dadurch gekennzeichnet,** daß die Optikführungshülse (5) mit einem Gewindeansatz (9a) und die Beleuchtungsbaugruppe (17) mit einer entsprechenden Gewindebohrung (21a) versehen ist, um diese Teile drehsicher miteinander zu verbinden.

## Claims

1. An endoscope, more particularly an arthroscope, comprising an illumination component (17) and an observation component (1) which may be interconnected, the observation component (1) having an optics-carrying shaft (2) which may be pushed inside a channel (18) of the illumination component (17), characterised in that the optics-carrying shaft (2) is part of a rotary optics (3) which is surrounded by an optics-guide sleeve (5), the rotary optics (3) being rotatably or displaceably mounted inside the optics-guide sleeve (5) and that the optics-guide sleeve (5) has, at one end, a coupling means (9) via which it may be connected to the illumination component (17) and, at the other end, a detachable optical-funnel (12) near the eyepiece.

2. An endoscope according to claim 1, characterised in that the coupling means (9) is in the form of a screw connection.

3. An endoscope according to claim 2, characterised in that the optics-guide sleeve (5) has a thread connection (10) with an outer thread (11) for the optical funnel (12) and that said optical funnel has, in turn, an attachment (13) with a corresponding inner thread (14).

4. An endoscope according to claim 3, characterised in that the optical funnel (12) and an attachment socket (24) of a recording unit (23) may be insertingly and/or clampingly interconnected.

5. An endoscope according to claim 4, characterised in that the optical funnel (12) has an annular groove (16a) or an annular seam (16b) on its outer surface (15) and may be inserted or clicked into the attachment socket (24) which has a corresponding (counter) annular seam (26b) or groove (26a) on its inner surface (25).

6. An endoscope according to claim 5, characterised in that a sterile cover (27) enveloping the recording unit (23) may be fixed to the attachment socket (24) which forms an adaptor, the connecting point between the attachment socket (24) and optical funnel (12) being located outside the cover (27).

7. An endoscope according to one of the preceding claims, characterised in that the optics-guide sleeve (5) forms a substantially closed chamber in which a rotational ring (4) is arranged in the form of a manual operating means for turning the rotary optics (3) so as to be accessible from outside, the optics-guide sleeve (5), which is fixed with respect to the rotary optics (3), having at one of its ends connecting means for the attachment socket (24) which is also similarly fixed and forms a video adaptor, and having at its other end a sleeve for protecting the optics-carrying shaft (2) of the rotary optics (3), or connecting means for this.

8. An endoscope according to claim 7, characterised in that the rotational position of the rotary optics (3), relative to the optics-guide sleeve (5), may be adjusted by means of a rotational ring (4).

9. An endoscope according to claim 8, characterised in that the optics-guide sleeve (5) has, in its wall, at least one recess (8) through which the rotational ring (4) of the rotary optics (3), which is especially in the form of a knurled ring, may be actuated.

10. An endoscope according to one of the preceding claims, characterised in that the optical funnel (12) closes a removal aperture of the optics-guide sleeve (5) through which the rotary optics (3) may be removed from the optics-guide sleeve (5).

11. An endoscope according to one of the preceding claims, comprising an illumination component (17) and an observation component (1) which may be locked, relative to one another, the observation component (1) having an optics-carrying shaft (2) which may be inserted inside a channel (18) of the illumination component (17), characterised in that the optics-guide sleeve (5) is provided with a threaded attachment (9a) and the illumination component (17) is provided with a corresponding threaded bore (21a) for the antirotational connection of said parts.

## Revendications

1. Endoscope, en particulier arthroscope, constitué par un bloc d'éclairage (17) et un bloc d'observation (1) qui peuvent être reliés l'un à l'autre, le bloc d'observation (1) comportant une tige (2) portant le système optique, qui peut être enfilée à l'intérieur d'une canule (18) du bloc d'éclairage (17), caractérisé en ce que la tige (2) portant le système optique fait partie d'une optique rotative (3) qui est entourée par une douille de guidage d'optique (5), l'optique rotative (3) étant montée en rotation et en déplacement à l'intérieur de la douille de guidage d'optique (5), et en ce que la douille de guidage d'optique (5) présente à une extrémité un dispositif d'accouplement (9) par l'intermédiaire duquel elle peut être reliée au bloc d'éclairage (17) et à l'autre extrémité, une trémie d'optique (12) amovible, du côté de l'oculaire.

2. Endoscope selon la revendication 1, caractérisé en ce que le dispositif d'accouplement (9) est réalisé sous la forme d'une liaison par vissage.

3. Endoscope selon la revendication 2, caractérisé en ce que la douille de guidage d'optique (5) présente un raccord fileté (10) avec filetage (11) pour la trémie d'optique (12) et en ce que celle-ci présente à son tour une embase (13) ayant un taraudage (14) correspondant.

4. Endoscope selon la revendication 3, caractérisé en ce que la trémie d'optique (12) et une tubulure de raccordement (24) d'une unité d'enregistrement (23) peuvent être reliées l'une à l'autre par enfichage et/ou par serrage.

5. Endoscope selon la revendication 4, caractérisé en ce que la trémie d'optique (12) est pourvue sur sa face extérieure (15) d'une gorge annulaire (16a) ou d'un bourrelet annulaire (16b) et peut être enfichée ou encliquetée dans la tubulure de raccordement (24) qui présente sur sa surface intérieure (25) un bourrelet annulaire (26b) ou une gorge annulaire (26b) complémentaires.

6. Endoscope selon la revendication 5, caractérisé en ce qu'une housse (27) stérile enveloppant l'unité d'enregistrement (23) peut être fixée sur une tubulure de raccordement (24) formant un adaptateur, l'emplacement de liaison entre la tubulure de raccordement (24) et la trémie d'optique (12) se trouvant en dehors de la housse (27).

7. Endoscope selon l'une quelconque des revendications précédentes, caractérisé en ce que la douille de guidage d'optique (5) forme une chambre sensiblement fermée dans laquelle une bague de rotation (4) formant moyen d'actionnement manuel pour tourner l'optique rotative (3) est agencée de telle sorte qu'elle est accessible depuis l'extérieur, la douille de guidage d'optique (5) stationnaire par rapport à l'optique rotative (3) présentant à l'une de ses extrémités des moyens de raccordement pour la tubulure de raccordement (24) également stationnaire formant un adaptateur vidéo, et à l'autre extrémité une douille pour protéger la tige (2) portant le système optique de l'optique rotative (3) ou des moyens de raccordement à cet effet.

8. Endoscope selon la revendication 7, caractérisé en ce que la position de rotation relative de l'optique rotative (3) par rapport à la douille de guidage d'optique (5) est réglable au moyen d'une bague de rotation (4).

9. Endoscope selon la revendication 8, caractérisé en ce que la douille de guidage d'optique (5) présente dans sa paroi au moins un évidement (8) à travers lequel la bague de rotation (4) de l'optique rotative (3) - réalisée en particulier sous forme d'une bague moletée - peut être actionnée.

10. Endoscope selon l'une quelconque des revendications précédentes, caractérisé en ce que la trémie d'optique (12) ferme une ouverture de retrait de la douille de guidage d'optique (5) à travers laquelle l'optique rotative (3) peut être extraite hors de la douille de guidage d'optique (5).

11. Endoscope selon l'une quelconque des revendications précédentes, constitué par un bloc d'éclairage (17) et par un bloc d'observation (1), qui peuvent être arrêtés l'un par rapport à l'autre, le bloc d'observation (1) présentant une tige (2) portant le système optique, qui peut être enfilée à l'intérieur d'une canule (18) du bloc d'éclairage (17), caractérisé en ce que la douille de guidage d'optique (5) est pourvue d'un épaulement fileté (9a), et en ce que le bloc d'éclairage (17) est pourvu d'un taraudage (21a) correspondant pour relier ces pièces l'une à l'autre pour empêcher qu'elle tournent.
